# EUROPEAN PATENT APPLICATION

(11) **EP 3 520 648 A1**
(43) Date of publication of application: **07.08.2019**
(21) Application number: 17855553.8
(22) Date of filing: 31.08.2017
(51) Int. Cl.: A45D 44/22, G06T 1/00, A61K 8/84

(54) **THIN FILM AND THIN FILM FORMATION METHOD**

(30) Priority: 27.09.2016 JP 2016187857
(71) Applicant: Panasonic Intellectual Property Management Co., Ltd., Osaka-shi, Osaka 540-6207 (JP)
(72) Inventor: SHINODA, Masayo, Osaka-shi, Osaka 540-6207 (JP)
(74) Representative: Grünecker Patent- und Rechtsanwälte PartG mbB
(86) International application number: PCT/JP2017/031298
(87) International publication number: WO 2018/061595

(57) **Abstract**

A structure is realized where effects imparted to an applied face can be improved. A thin film that is applied to an applied face of an application object has front and back faces, of which one is disposed at the applied face side in use, and includes a thin film main member that has biocompatibility, a first functional layer including a first functional component laminated on a front face of the thin film main member, and a second functional layer including a second functional component that differs from the first functional component, layered on a back face of the thin film main member.

## Description

### Technical Field

The present invention relates to a thin film such as a beauty sheet that can be applied to the skin, for example, and to a thin film forming method.

### Background Art

There has been known heretofore thin films such as a beauty sheet or the like, which can be applied to the skin, and to which a functional component has been added, such as a cosmetic substance, medical substance, or the like (e.g., see PTL 1). The forming method of the thin film described in PTL 1 involves printing an image portion, including a functional component that has a predetermined shape, size, and color, on a thin film, to make discolorations of the skin such as blemishes less conspicuous. The user then applies the image portion of the thin film to the target position, which enables facial makeup and skin care such as masking blemishes to be easily performed.

### Citation List

### Patent Literature

PTL 1: Japanese Unexamined Patent Application Publication No. 2015-43836
PTL 2: Japanese Unexamined Patent Application Publication No. 2012-86475
PTL 3: Japanese Unexamined Patent Application Publication No. 2015-117196
PTL 4: Japanese Unexamined Patent Application Publication No. 2016-88882
PTL 5: Japanese Unexamined Patent Application Publication No. 10-46073
PTL 6: Japanese Unexamined Patent Application Publication No. 2005-126729
PTL 7: Japanese Unexamined Patent Application Publication No. 2012-203425

### Summary of Invention

Now, in the case of the thin film described in PTL 1, the image portion is only formed on one side of the thin film, so the effects that the thin film have on the applied face may be limited.

An aspect of the present disclosure provides a thin film that can improve effects on the applied face, and a thin film forming method.

The thin film according to the present disclosure is a thin film that is applied to an applied face of an application object has front and back faces, of which one is disposed at the applied face side in use, and includes a thin film main member that has biocompatibility, a first functional layer including a first functional component laminated on a front face of the thin film main member, and a second functional layer including a second functional component that differs from the first functional component, layered on a back face of the thin film main member.

According to an aspect of the present disclosure, a thin film that can improve effects on the applied face, and a thin film forming method, can be provided.

### Brief Description of Drawings

Fig. 1A is a cross-sectional schematic diagram of a thin film according to a first embodiment according to the present disclosure.
Fig. 1B is a schematic diagram illustrating Fig. 1A in a state as viewed from above.
Fig. 1C is a schematic diagram illustrating Fig. 1A in a state as viewed from below.
Fig. 2 is a schematic diagram illustrating an example of a makeup support system used to form the thin film in the first embodiment.
Fig. 3 is a block diagram of an image processing device making up the makeup support system.
Fig. 4 is a flowchart of a thin film forming method for forming the thin film according to the first embodiment.
Fig. 5A is a cross-sectional schematic diagram of a thin film according to a second embodiment according to the present disclosure.
Fig. 5B is a schematic diagram illustrating Fig. 5A in a state as viewed from above.
Fig. 5C is a schematic diagram illustrating Fig. 5A in a state as viewed from below.
Fig. 6A is a cross-sectional schematic diagram of a thin film according to a third embodiment according to the present disclosure.
Fig. 6B is a schematic diagram illustrating Fig. 6A in a state as viewed from above.
Fig. 6C is a schematic diagram illustrating Fig. 6A in a state as viewed from below.
Fig. 7A is a cross-sectional schematic diagram of a thin film according to a fourth embodiment according to the present disclosure.
Fig. 7B is a schematic diagram illustrating Fig. 7A in a state as viewed from above.
Fig. 7C is a schematic diagram illustrating Fig. 7A in a state as viewed from below.

### Description of Embodiments

Embodiments of the present disclosure will be described in detail with reference to the drawings.

### [1. First Embodiment]

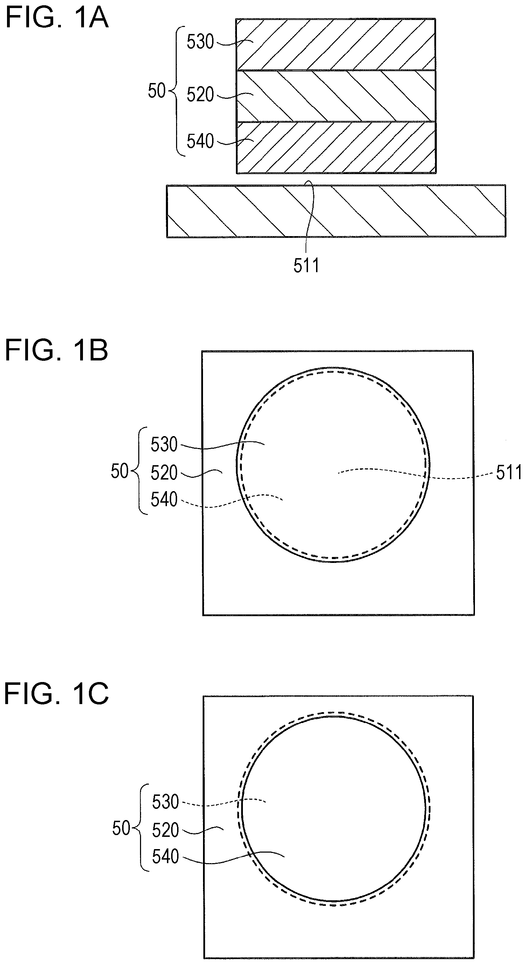

A thin film 50 according to a first embodiment of the present disclosure will be described with reference to Figs. 1A through 1C.

The thin film 50 includes a thin film main member 520 in the shape of a sheet, a first functional layer 530 in the shape of a sheet that is laminated on the front face of the thin film main member 520, and a second functional layer 540 in the shape of a sheet that is laminated on the front face of the thin film main member 520.

The thin film main member 520 serves as a supporting base for the first functional layer 530 and the second functional layer 540. The first functional layer 530 and second functional layer 540 impart effects that are cosmetic, medical, or the like, for example, to a discoloration region 511 that is an applied face, in a state of having been applied to the discoloration region 511 (hereinafter referred to as applied state). The thin film 50 is applied, manually or by device, so that the portion where the first functional layer 530 and second functional layer 540 are formed overlays the discoloration region 511 in the front-back direction. Note that the applied face is not restricted to the discoloration region 511, and may be various types of regions, such as a wrinkle region where wrinkles or the like are formed on the face of the user, an injury region where there is a scar or the like, and so forth, for example.

Note that in the following description, the face of the thin film 50 that is on the side applied in contact with the discoloration region 511 (e.g., a blemish on the skin of the user) will be referred to as "skin-side face", and the opposite side face will be referred to as "outer-side face". The front face of the thin film 50 according to the present embodiment may be used as the skin-side face, or the front face may be used as the outer-side face. Also, the term "front-back direction" in the following description means the front-back direction of the thin film main member 520.

A specific configuration of the thin film 50 according to the present embodiment will be described below. The thin film main member 520 is a sheet member that is not unnatural when applied to human skin, is transparent, skin colored, white, or translucent, and also has biocompatibility. The thickness of the thin film main member 520 used in this way may be 10 to 10,000 nm (10 nm to 10 µm), and particularly preferably is 10 to 1000 nm. In a case where the thin film main member has hydrophobicity, 10 to 800 nm is preferable in particular. The shape of the thin film main member 520 illustrated in Figs. 1B and 1C is rectangular in plane view. Note however, that various shapes of the thin film main member 520 in plane view may be employed, in accordance with the shape of the discoloration region 511 and the shape at the periphery of the discoloration region 511. The thin film main member 520 may be any of sheets formed by spin coating, roll-to-roll processing, the LB process, or the like, fiber sheet forms where fibers generated by electrospinning or the like lie one on another, and further may be any porous form formed by phase separation, drawing, etching or the like, and preferably has permeability to allow permeation of the functional component of the functional layer on at least the outer-side face. The thin film main member 520 may have optional slits at the periphery or in plane, so as to be able to conform to the shape of the discoloration region 511 or the periphery of the discoloration region 511.

Preferable examples of the material of the thin film main member 520 include polyglycolic acid, polylactic acid, polycaprolactone, polyethylene succinate, polyethylene terephthalate, polyethylene glycol, polypropylene glycol, nylon, polyglutamic acid, polyimide, acrylic silicone, trimethylsiloxysilicic acid, dimethylpolysiloxane, acrylate-silicone copolymer, alkyl acrylate-amide copolymer, polyethylene, polypropylene, polyurethane, and like resins, polyaniline, polythiophene, polypyrrole, polyvinyl alcohol, polycarbonate, polystyrene, Nafion, and like polymers, and pullulan, cellulose (e.g., carboxymethyl cellulose, hydroxyethyl cellulose, etc.), starch, chitin, chitosan, alginic acid, corn starch, pectin, arabinoxylan, glycogen, amylose, amylopectin, hyaluronic acid, and like polysaccharides. Particularly preferable as materials for the thin film main member 520 are polylactic acid, cellulose (e.g., carboxymethyl cellulose, hydroxyethyl cellulose, etc.), starch, chitin, chitosan, alginic acid, corn starch, and polyurethane. Other materials that can be employed in the thin film main member are described in PTL 2, for example, so detailed description here will be omitted.

The first functional layer 530 has a form of a sheet that has been laminated on the front face of the thin film main member 520 (lower-side face in Fig. 1A). This first functional layer 530 is formed by printing a first printing material including a first functional component on the front face of the thin film main member 520. Note that the first functional layer 530 may be a single sheet, or may be a laminated member where multiple sheets are laminated. The first functional component may be of a single type, or of multiple types. The first functional component is, for example, a color component, a shielding component, a whitening component, an ultraviolet ray protection component (ultraviolet ray absorbing component, ultraviolet ray scattering component), a moisturizing component, an anti-inflammatory component, an antibacterial component, an reactive oxygen suppressing component, an antiaging component, a blood circulation promoting component, or the like. Other functional components may be various types of functional components, such as a component to improve skin resilience and elasticity, a component to promote activation of cells, and so forth.

Note that each of the first and second functional layers may further be layers where multiple types of components have been mixed. Also, the first and second functional layers may each be configured of further multiple layers, configured with different components laminated as different layers. For example, a part of layers of the first or second functional layers may have makeup functions such as a foundation layer and ultraviolet ray protection layer, while another layer (a layer coming into contact with the skin) has skincare functions. Also, a part of layers of the first or second functional layers may, for example, be designed with layers having different light reflection or refraction, so that sensuous luster or matte impressions are designed, not just functionality. Alternatively, in the first or second functional layers, a layer closer to the thin film main member may be made up of a component that has a faster permeation rate than an outer side layer. The first functional component of the first functional layer 530 may be of a nature of passing through the thin film main member 520, or of a nature of not passing through. Also, the first functional component may have a nature where the rheology changes depending on temperature or on whether moisture is present or not. Note that the various types of components mentioned above are described in PTL 3 and 4, for example, so detailed description will be omitted.

Note that the first functional layer 530 illustrated in Figs. 1B and 1C is formed in a circular shape in plane view. However, various types of shapes can be employed for the shape of the first functional layer 530 in plane view. Note that the cross-sections of the functional layers are not restricted, and may be porous forms, forms where fibers lie one on another, high-density faces with no mesh or holes, and so forth. According to this configuration, the effects of ointments or medicinal components applied beforehand being retained more readily, moved less readily, and rubbed less readily, are higher as compared to a case of configuring by thin film alone.

The second functional layer 540 has a form of a sheet that has been laminated on the back face of the thin film main member 520 (upper-side face in Fig. 1A). This second functional layer 540 is formed by printing a second printing material including a second functional component on the back face of the thin film main member 520. The second functional component may be of a single type, or of multiple types. This second functional component includes at least one type of functional component that differs from the first functional component. In other words, the second functional component includes at least one type of functional component that exhibits effects (e.g., effects cosmetically or medicinally) different from the first functional component. All of the second functional component may different from the first functional component. The aforementioned various types of functional components can be employed for the second functional component, in the same way as in the first functional component. The second functional component of the second functional layer 540 may be of a nature of passing through the thin film main member 520, or of a nature of not passing through. Note that the second functional layer 540 illustrated in Figs. 1B and 1C is formed in a circular shape having a diameter smaller than the first functional layer 530, in plane view. In the case of the present embodiment, the entirety of the second functional layer 540 overlays the first functional layer 530 with regard to the front-back direction, as illustrated in Fig. 1C. Note however, that various types of shapes can be employed for the shape of the second functional layer 540 in plane view.

The thin film 50 according to the present embodiment, configured as described above, is applied to the skin of the user, so that the second functional layer 540 overlays the discoloration region 511, with the back face as the skin-side face as illustrated in Fig. 1A, for example. Accordingly, the shapes of the first functional layer 530 and second functional layer 540 are decided appropriately, in accordance with the size, shape, and position of the discoloration region 511. The functional components making up the first functional layer 530 and second functional layer 540 are decided as appropriate in accordance with the effects desired to be imparted to the discoloration region 511.

Now, in a case where the back face is the skin-side face, for example, if the first functional component is made to be of a nature that passes through the thin film main member 520, the first functional component of the first functional layer 530 can be made to permeate the discoloration region 511 at a later timing than the second functional component of the second functional layer 540 permeating the discoloration region 511. Using such natures enables the relation between the timing of the effects of the first functional layer 530 acting on the discoloration region 511 and the timing of the effects of the second functional layer 540 acting on the discoloration region 511 to be adjusted.

In a case where there is a difference in transpiration rate between the first functional component of the first functional layer 530 and the second functional component of the second functional layer 540 for example, the functional component of the functional layer at the skin-side face side (in the case of the present embodiment, the second functional component of the second functional layer 540) is a functional component with a higher transpiration rate. According to this arrangement, the effects of the functional component with a high transpiration rate can be exhibited in a high-concentration state, without waste.

### [1.2 System Configuration]

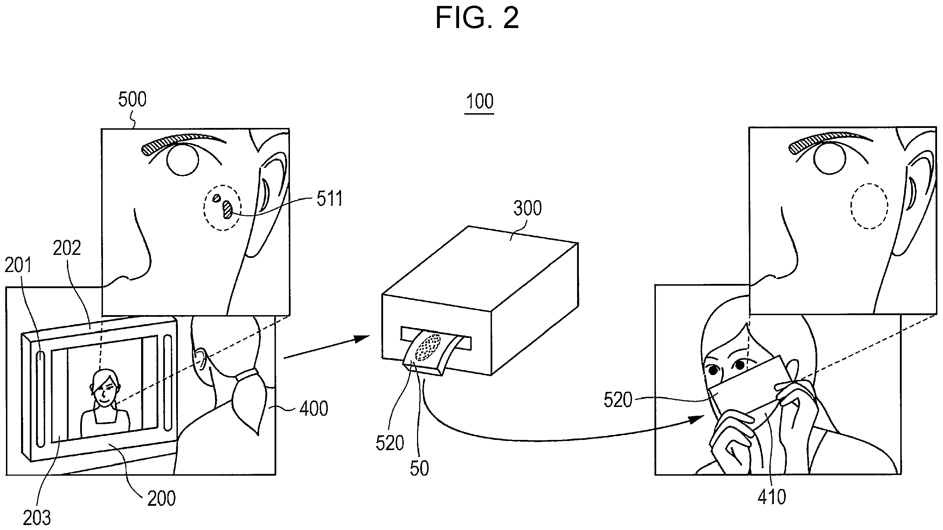

The overview of an example of a makeup support system used in forming the thin film 50 according to the first embodiment will be described with reference to Fig. 2.

Fig. 2 is a schematic diagram illustrating the overview of an example of the makeup support system.

In Fig. 2, the makeup support system 100 includes an image processing device 200 that has an illumination unit 201, a camera 202, and a display unit 203 such as a touchscreen liquid crystal display or the like, and a printing device 300 that is communicably connected to the image processing device 200. The image processing device 200 and printing device 300 are installed in, for example, factories, cosmetics shops, beauty shops, medical institutions, makeup rooms for grooming, event sites, private residences, and so forth. Note that the image processing device 200 may be a portable device that is readily carried.

In a state where the face of a user 400 situated to the front of the display unit 203 is illuminated by the illumination unit 201, the image processing device 200 takes an image of the face by the camera 202 disposed nearby the display unit 203. The image processing device 200 the displays an image where the taken image has been inverted horizontally (hereinafter referred to as "facial image") 500 on the display unit 203. That is to say, the image processing device 200 is configured to give the user 400 a sensation close to that of looking into a mirror.

The image processing device 200 also has a function of extracting information relating to the discoloration region 511 (hereinafter referred to as discoloration region information) from the obtained facial image 500 (or from the image before inversion). Specifically, the image processing device 200 analyzes color difference within the facial image 500, and reflection and absorption of visible light illumination by which the user is illuminated when the image is being taken, and determines the discoloration region 511, for example. The discoloration region information includes shape information containing at least one type of information of position, size, range, and shape, color information, classification information of the discoloration region, and so forth, of the discoloration region 511, for example. The classification information of the discoloration region includes, based on difference of cause of discoloration at the discoloration region 511, the classifications of pigmented spots, chloasma, nevus spilus, melanocytic nevus, nevus of Ota, acquired dermal melanocytosis, erythema, purpura, vitiligo, bruises, moles, darkening of pores, sunburn areas, acne, acne scars, pigmentation due to friction or inflammation, wrinkles, freckles, tattoos, warts, scarring, and so forth. Note that the image processing device 200 has functions of obtaining information of the applied face equivalent to the aforementioned discoloration region information, in a case where the applied face is other than the discoloration region 511, as well. In the following description, the applied face is the discoloration region 511.

The image processing device 200 has a function of extracting information of the peripheral regions of the discoloration region 511 (hereinafter referred to as peripheral region information). The peripheral region information includes, for example, color information relating to the color at the periphery of the discoloration region 511 (hereinafter referred to as peripheral color), and shape information relating to shape, such as skin contour or the like. Note that that peripheral color may be decided by the image processing device 200 based on measurement values of color information such as a comprehensive region or the like obtained by an external measurement device such as a spectral colorimeter or the like, or may be decided by being selected from color samples prepared beforehand. Alternatively, color information of foundation or concealer or the like that the user likes may be obtained by the image processing device 200, and decided by the image processing device 200 to be the peripheral color.

The image processing device 200 also generates print data based on the discoloration region information and peripheral region information. The print data includes data relating to the printing material (hereinafter referred to as material data) and image data, for example. The material data is decided based on classification information of the discoloration region information. For example, the image processing device 200 selects functional components effective regarding the discoloration region 511 as print data, such as whitening components, vitamins, or the like, based on the discoloration region.

Additionally, the image processing device 200 can generate print data based on information other than the discoloration region information and peripheral region information. For example, wrinkles, smile lines, mid-cheek lines, bunny lines, and so forth (hereinafter referred to as wrinkles and so forth) in the facial image 500 are detected. In a case where the object is to make these wrinkles and so forth inconspicuous, the image processing device 200 selects a functional component having moisturizing effects as the print data.

The following is a brief description of the functions of the image processing device 200 that are necessary when forming the thin film 50 according to the first embodiment that has been described above. Note that in the following description, the side of the thin film 50 where the second functional layer 540 has been formed on the skin-side face.

When forming the thin film 50, the image processing device 200 generates print data for the first functional layer 530 and the second functional layer 540. The image processing device 200 decides material data relating to the first printing material for the first functional layer 530, based on the classification information of the discoloration region information. The image processing device 200 also decides image data relating to the first functional layer 530, based on the shape information of the discoloration region information. The image processing device 200 generates the pint data for the second functional layer 540 in the same way.

For example, if the first functional layer 530 is to be imparted blemish-masking functions, a color component is selected as the first functional component. Specifically, the image processing device 200 decides a color component that will blend in with the peripheral color in a state where the first functional layer 530 is applied to the discoloration region 511, based on the peripheral information.

The image processing device 200 also decides image data relating to the position on the thin film main member 520 (e.g., coordinates) dimensions shape, color, and so forth, with regard to the first functional layer 530 and second functional layer 540. The image processing device 200 then outputs print data including the material data and image data to the printing device 300.

The printing device 300 prints images on the thin film main member 520 of the thin film 50, based on the print data output from the image processing device 200. This printing device 300 has functions of, for example, duplex printing where the first functional layer 530 is printed on the front face of the thin film main member 520 and thereafter the second functional layer 540 is printed on the back face of the thin film main member 520. The printing device 300 also has a heater function to dry the first functional layer 530 after having printed the first functional layer 530 on the front face of the thin film main member 520. Note that the duplex printing functions of the printing device 300 are almost the same as duplex printing functions of conventionally-known printing devices and so forth, so detailed description will be omitted.

The printing device 300 then outputs the thin film 50. The user 400 applies the thin film 50 to the discoloration region 511 so that the second functional layer 540 overlays the discoloration region 511 in the front-back direction, with the side of the thin film 50 on which the second functional layer 540 has been formed as the skin-side face.

The second functional component included in the second functional layer 540 imparts predetermined effects to the discoloration region 511 in the applied state. On the other hand, the first functional layer 530 is not directly applied to the discoloration region 511, but imparts predetermined effects to the discoloration region 511 based on the first functional component (e.g., effects of masking blemishes). Thus, the makeup support system 100 enables predetermined treatment of the discoloration region 511 through the simple and time-saving action of applying the thin film 50 to the skin 410.

Now, printing materials (ink) other than the functional components used for printing by the printing device 300, and specific configurations of each part, are described in PTL 5 through 7 for example, so detailed description thereof will be omitted here.

### [1.2.1 Notes Regarding Image Processing Device 200]

The image processing device 200 may have a function where, in a case that the current measurement information has changed in comparison with past measurement information, such as the skin becoming darker or the like, for example, determination is made that there is a possibility of sunburn, and guidance such as "sunburn care is advised" is displayed on the display unit 203. At this time, the image processing device 200 prompts the user to select whether or not to perform sunburn care. In a case where the user has selected to execute sunburn care, a screen to select from recommended functional components is displayed on the display unit 203. The user can order a thin film with the selected functional component applied via the Internet, which will be delivered to the user at a later date by home delivery. Alternatively, if the user has a printer for printing the thin film at home, the user can generate the thin film on demand. Thus, the image processing device 200 can also decide print data for thin films based on input from the user.

The image processing device 200 may have a function where, in a case that no effects of improvement regarding blemishes, wrinkles, smile lines, and so forth are observed in comparison of past measurement information with current information, for example, adjustment is performed such as increasing the concentration of the functional component that the functional layers include per unit of area.

The image processing device 200 also has a function extracting facial parts from feature points on the face, and deciding thin film forms in accordance with the facial parts. The image processing device also has a function of including information of slits to be formed at the periphery and in plane of the thin film in the print data, to allow conforming to the contour of the facial part.

The image processing device 200 also has a function where, in a case that the areas to be treated are broad ranges, the thin film is generated in accordance with the facial parts. This facilitates handling by the user, and enables easy comprehension of positions to be applied. That is to say, the image processing device 200 has a function where, in a case that discoloration regions and wrinkles and so forth are many and far apart, and the areas to be treated are broad ranges, decision is made between a case of performing care with a single thin film, and a case of performing care with multiple thin films.

The image processing device 200 may have a function where, in a case that a part where the size of the discoloration region 511 is not readily accurately obtained from a two-dimensional taken image (e.g., around the temples, etc.), the size of the discoloration region 511 is calculated taking the average curvature of the face into consideration.

### [1.3 Thin Film Forming Method]

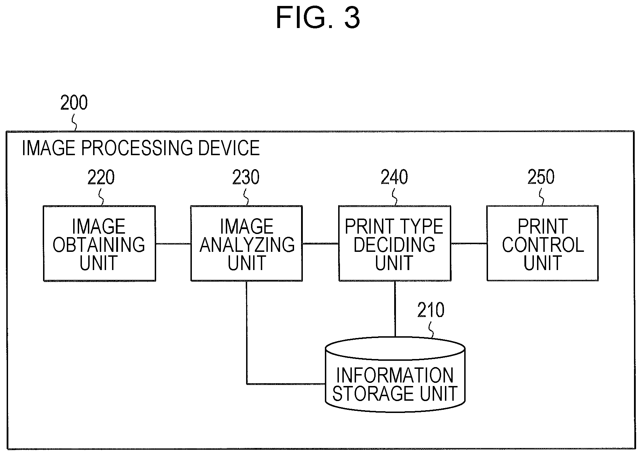

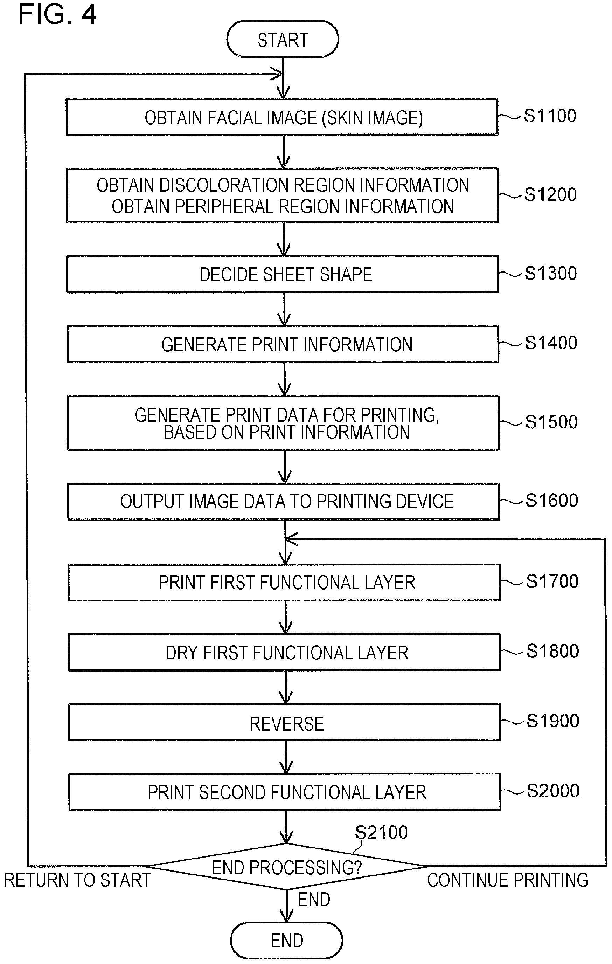

An example of the thin film forming method to form the thin film 50 according to the first embodiment will be described with reference to Figs. 1A through 4. Note that Fig. 3 is a block diagram of the image processing device 200 making up the makeup support system, and Fig. 4 is a flowchart of the thin film forming method according to this example. A case where the side of the thin film 50 on which the second functional layer 540 is formed is the skin-side face, as illustrated in Fig. 1A, will be described below. Description will be made regarding an arrangement where the first functional component of the first functional layer 530 is a color component for masking blemishes, and the second functional component of the second functional layer 540 is a whitening component.

### [1.3.1 First Step]

First, in step S1100, an image obtaining unit 220 of the image processing device 200 obtains a facial image 500 (see Fig. 2).

### [1.3.2 Second Step]

Next, in step S1200, an image analyzing unit 230 of the image processing device 200 obtains discoloration region information relating to a discoloration region 511, and peripheral region information relating to peripheral regions of the discoloration region 511, from the facial image 500. At this time, the image analyzing unit 230 obtains various types of information necessary for image analysis, stored in an information storage unit 210. Note that the information storage unit 210 stores beforehand various types of information necessary for deciding printing type by a print type deciding unit 240, as well. The information included in discoloration region information and peripheral region information is as described above.

An example of a method of obtaining color information for the discoloration region information will be described.

For example, in order to obtaining color information of the discoloration region 511, the image analyzing unit 230 first obtains a region of a color region determined beforehand, from a region excluding facial part which are nostrils, eyes, mouth, eyebrows, and so forth, as a skin region. Next, the image analyzing unit 230 classifies the obtained skin region into discoloration region 511 and non-discoloration region, with a predetermined pixel value such as brightness as a boundary, for example, thereby extracting the discoloration region 511. Note that at this time, the image analyzing unit 230 may handle discoloration regions 511 with an area equal to or smaller than a predetermined area as non-discoloration regions. The image analyzing unit 230 then extracts the average value of a non-discoloration region nearby the discoloration region 511 as a peripheral color. The image analyzing unit 230 may extract the discoloration region 511 and peripheral color by a technique described in PTL 1.

### [1.3.3 Third Step]

Next, in step S1300, the image analyzing unit 230 decides the shape of the sheet from the layout of facial parts. Specifically, the image analyzing unit 230 decides a closed shape that can cover the entirety of one of more extracted discoloration regions 511 in the form of a sheet, while avoiding the positions of facial parts. The sheet shape is equivalent to the shape of the thin film main member 520 of the thin film 50 in the case of the above-described first embodiment. In a case where the applied face is part of the face, the size of the sheet is preferably restricted to 5 cm × 5 cm or smaller, or the like, since the face has three-dimensional features while the basic sheet shape is flat.

### [1.3.4 Fourth Step]

Next, in step S1400, the print type deciding unit 240 of the image processing device 200 generates print information based on the discoloration region information and peripheral region information obtained in step S1200. The print information includes, for example, information of images, printing material, whether or not to use a base material, and whether or not a printing process.

In the case of this example, the print type deciding unit 240 generates print information relating to the first functional layer 530 of the thin film 50 based on the discoloration region information and peripheral region information. Specifically, in the case of this example, print information is generated so that the first functional layer 530 exhibits effects of masking blemishes with regard to the discoloration region 511, taking into consideration the dimensions, shape, and color of the discoloration region 511 included in the discoloration region information, and the peripheral color included in the peripheral region information and so forth. That is to say, the print type deciding unit 240 sets a color component that exhibits the functions of masking blemishes, as the first functional component of the first functional layer 530, in the print information.

Next, the print type deciding unit 240 generates print information for the second functional layer 540 of the thin film 50, based on the discoloration region information. Specifically, in the case of this example, print information is generated so that the second functional layer 540 can exhibit whitening effects on the discoloration region 511, based on the dimensions, shape, and color and so forth of the discoloration region 511 included in the discoloration region information. The print type deciding unit 240 then outputs the print information to a print control unit 250.

The print type deciding unit 240 can also display information relating to the first functional layer 530 or second functional layer 540 generated based on the print information, on the display unit 203. For example, the print type deciding unit 240 has a function of displaying on the display unit 203 a simulation image, where an image of the first functional layer 530 or second functional layer 540 generated based on print information is overlaid on the discoloration region 511 of the facial image 500. The user can adjust print information relating to the position, size, range, shape color, and so forth, for the first functional layer 530 or second functional layer 540, while observing the simulation image displayed on the display unit 203.

### [1.3.5 Fifth Step]

Next, in step S1500, the print control unit 250 of the image processing device 200 generates print data of contents to be printed, at portions corresponding to the first functional layer 530 on the front face of the thin film main member 520 and portions corresponding to the second functional layer 540 on the back face of the thin film main member 520, based on the print information generated in step S1400. The print data includes the above-described material data and image data. The print control unit 250 can also include control information instructing operation in accordance with the print information, in the print data. For example, control information can include an instruction to dry the first functional layer 530 between printing of the first functional layer 530 and printing of the second functional layer 540.

### [1.3.6 Sixth Step]

In step S1600, the print control unit 250 outputs the generated print data to the printing device 300. Note that the above-described processing of S1100 through S1600 is executed at the image processing device 200.

### [1.3.7 Seventh Step]

In step S1700, the printing device 300 prints the first functional layer 530 on the front face of the thin film main member 520, based on the print data received from the print control unit 250. Description of the structure of the first functional layer 530 will be omitted here, since already described above. Various types of printing methods can be employed, such as spray printing, inkjet printing, offset printing, and so forth.

### [1.3.8 Eighth Step]

Next, in step S1800, the printing device 300 drives a drying device (omitted from illustration) such as an internal heater or the like, based on the control information included in the print data. This drying device performs drying processing to dry the first functional layer 530 printed on the front face of the thin film main member 520. Examples of the drying device include drying devices that use hot air, far infrared rays, and so forth. Note that the drying device preferably has a structure where the first functional component of the first functional layer 530 is not denatured. In the case of this example, the color component that is the first functional component does not readily exhibit denaturing under heat. In a case where the first printing material for the first functional layer 530 has high drying properties, the printing device 300 does not have to perform the drying processing.

### [1.3.9 Ninth Step]

Next, in step S1900, the printing device 300 reverses the thin film main member 520. The structure for reversing the thin film main member 520 can be realized by a reversing mechanism of the like of a conventional printing device having duple printing functions, so detailed description will be omitted. Note that the printing device 300 does not have to reverse the thin film main member 520 depending on the structure of the printing device.

### [1.3.10 Tenth Step]

Next, in step S2000, the printing device 300 prints the second functional layer 540 on the back face of the thin film main member 520, based on the print data received from the print control unit 250. Note that description of the structure of the second functional layer 540 will be omitted here, since already described above. In the case of this example, the printing device 300 does not perform drying processing on the second functional layer 540 printed on the back face of the thin film main member 520. The reason is that the second functional component for the second functional layer 540 is a whitening component, and may be denatured by heat. That is to say, functional components that exhibit beauty effects or medicinal effects may be denatured by heat, so drying processing preferably is not performed after printing. Various types of printing methods can be employed, such as spray printing, inkjet printing, offset printing, and so forth. The printing methods of the first functional layer and the second functional layer may be the same, or may be a combination of different methods. For example, the first functional component may be applied by inkjet printing for the first functional layer of the front face, and the second functional component may be applied by spray printing for the second functional layer of the back face.

### [1.3.11 Eleventh Step]

Finally, in step S2100, the image obtaining unit 220 of the image processing device 200 determines whether or not ending of processing has been instructed by a user operation or the like. In a case where ending of processing has not been instructed, and the user has selected "continue printing", the image obtaining unit 220 returns the flow to step S1700 in response to the selection by the user. In a case where the user has selected "return to start", the flow returns to step S1100 in response to the selection by the user. Further, in a case where ending of the processing has been instructed, the image obtaining unit 220 ends the flow of processing.

According to the above-described flow, the image processing device 200 can automatically extract discoloration regions 511 of the face, decide print data based on discoloration region information and peripheral region information extracted from the discoloration regions 511, and generate a thin film 50 that efficiently covers the discoloration region 511, using the printing device 300.

### [1.4 Effects and Advantages]

According to the thin film 50 of the present embodiment, different effects can be imparted to a discoloration region 511 with a single thin film 50. That is to say, the thin film 50 is provided with the first functional layer 530 on the front face of the thin film main member 520 and also is provided with the second functional layer 540 on the back face in the same way, with the first functional component for the first functional layer 530 and the second functional component for the second functional layer 540 differing from each other. Accordingly, the thin film 50 can impart both effects based on the first functional component (e.g., effects of masking blemishes) and effects based on the second functional component (e.g., whitening effects), to the discoloration region 511.

Also, in a case where the first functional component of the first functional layer 530 is a makeup component, contact between the first functional layer 530 and the sebum on the skin of the user in the applied state is mitigated by the thin film main member 520, whereby makeup can be suppressed from coming off.

### [2. Second Embodiment]

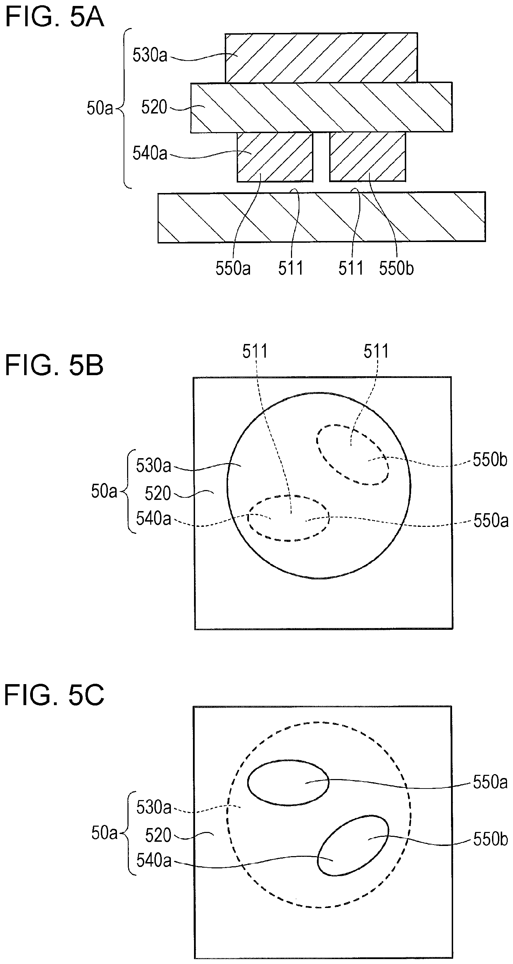

Thin film according to a second embodiment will be described with reference to Figs. 5A through 5C.

A thin film 50a according to the present embodiment has the thin film main member 520, a first functional layer 530a in sheet form that is laminated on the front face of the thin film main member 520, and a second functional layer 540a in sheet form that is laminated on the back face of the thin film main member 520 in the same say as the thin film 50 in the first embodiment. Note that the configuration of the thin film main member 520 is the same as in the thin film 50 according to the first embodiment.

The shape of the first functional layer 530a in plane view is the same as that in the first embodiment described above. In the case of the present embodiment, the first functional layer 530a imparts effects of masking blemishes as to the discoloration region 511. Accordingly, the first functional layer 530a is configured of a first functional component including at least a color component. This color component is the peripheral color, that is the color at the periphery of the discoloration region 511, for example. Note that the first functional layer 530a may have a gradient to match variance in colors at the periphery of the discoloration region 511, for example, which can improve blending of the first functional layer 530a to the skin color-wise. Thus, the first functional layer 530a has a size and shape overlaying two discoloration regions 511 formed on the skin and peripheral portions of the discoloration regions 511 in the front-back direction, in a state of being applied.

The second functional layer 540a is made up of a pair of back-side functional layer elements 550a and 550b, that are elliptic sheets laminated at two locations on the back face of the thin film main member 520. The pair of back-side functional layer elements 550a and 550b is configured of a second functional component including at least a shielding component that shields light. The pair of back-side functional layer elements 550a and 550b overlays the first functional layer 530a in entirety, in the front-back direction. In other words, the pair of back-side functional layer elements 550a and 550b is formed on the inner diameter side of the first functional layer 530a from the circumferential edge thereof, in plane view as illustrated in Fig. 5B.

The pair of back-side functional layer elements 550a and 550b is formed at positions on the thin film main member 520 so as to be capable of being applied overlaying the two discoloration regions 511 formed on the skin in the front-back direction. Note that in the case of this example, the pair of back-side functional layer elements 550a and 550b is formed having approximately the same shapes as the discoloration regions 511 overlaid in the applied state. However, the pair of functional layer elements 550a and 550b may be slightly larger in shape than the discoloration regions 511 which are overlaid in the applied state. Employing this configuration facilitates dealing with positional deviation when applying the thin film 50a. Other configurations are the same as in the first embodiment describe above. The forming method of the thin film 50a according to the present embodiment is almost the same as the forming method of the thin film described with reference to Fig. 4.

### [2.1 Notes]

### [2.2 Effects and Advantages]

According to the thin film 50a of the present embodiment, the effects of masking blemishes, which a single thin film 50a imparts to the discoloration regions 511 can be improved. That is to say, the shielding effect that the second functional layer 540a of the thin film 50a exhibits makes the discoloration regions 511 less likely to show through in a state where the thin film 50a is applied to the discoloration regions 511. As a result, the effects of blemish masking that the first functional layer 530a exhibits can be improved.

### [3. Third Embodiment]

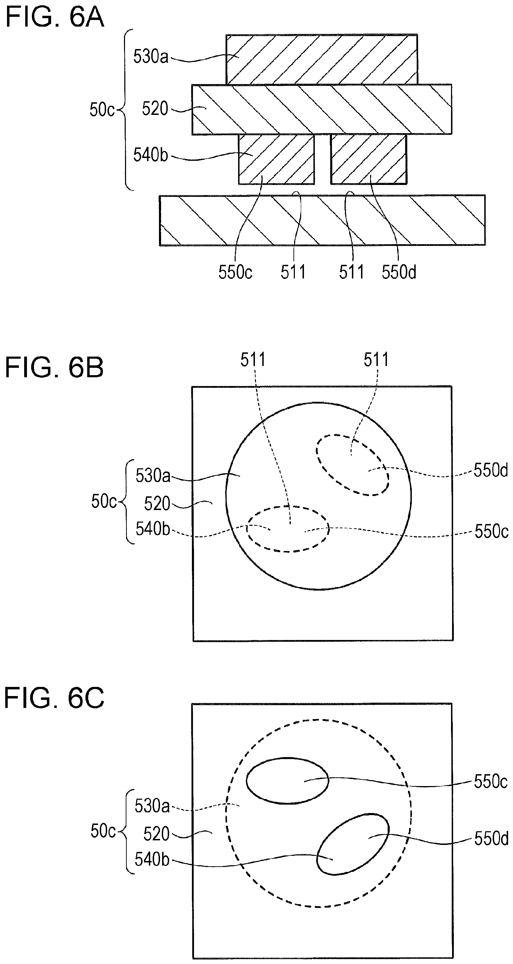

Thin film according to a third embodiment will be described with reference to Figs. 6A through 6C.

A thin film 50c according to the present embodiment has the thin film main member 520, a first functional layer 530a in sheet form that is laminated on the front face of the thin film main member 520, and a second functional layer 540b in sheet form that is laminated on the back face of the thin film main member 520, in the same way as the thin film 50a according to the second embodiment. Note that the configuration of the thin film main member 520 and the first functional layer 530a is the same as in the thin film 50a according to the second embodiment.

The second functional layer 540b is made up of a pair of back-side functional layer elements 550c and 550d, that are elliptic sheets laminated at two locations on the back face of the thin film main member 520, in the same way as the above-described second embodiment. The pair of back-side functional layer elements 550c and 550d is configured of a second functional component including a whitening component. Other configurations are the same as with the above-described second embodiment. The forming method of the thin film 50a according to the present embodiment is almost the same as the forming method of the thin film described with reference to Fig. 4.

### [3.1 Effects and Advantages]

According to the thin film 50c of the present embodiment, different effects can be imparted to the discoloration regions 511 with a single thin film 50c. That is to say, the thin film 50c can impart both blemish masking effects based on the first functional component and whitening effects based on the second functional component to the discoloration regions 511.

Also, in the applied state, the second functional layer 540b overlays only the discoloration regions 511 or the discoloration regions 511 and the periphery thereof very close to the discoloration regions 511. Accordingly, the thin film 50c does not impart whitening effects to non-discoloration regions that do not need whitening effects, and can concentrate whitening effects on the discoloration regions 511 where necessary.

### [4. Fourth Embodiment]

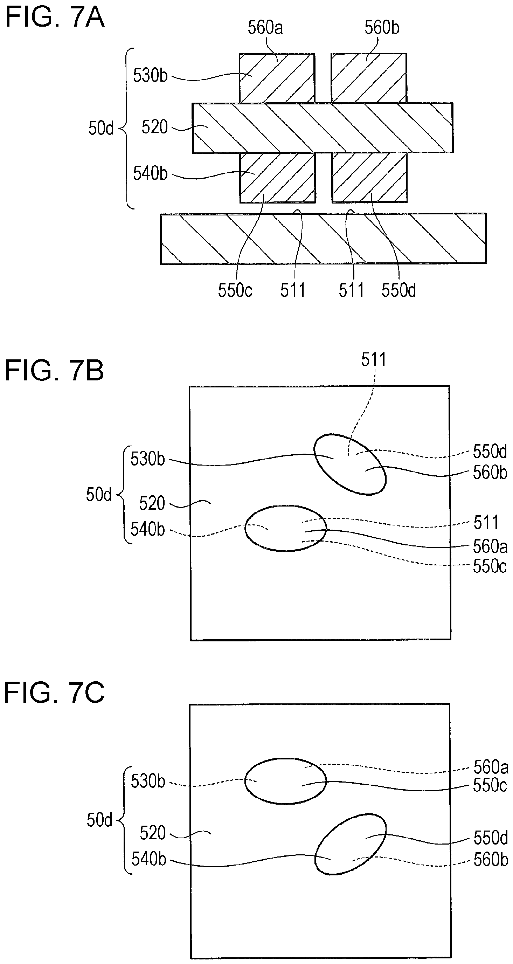

Thin film according to a fourth embodiment will be described with reference to Figs. 7A through 7C.

A thin film 50d according to the present embodiment has the thin film main member 520, a first functional layer 530b in sheet form that is laminated on the front face of the thin film main member 520, and a second functional layer 540b in sheet form that is laminated on the back face of the thin film main member 520. Note that the configuration of the thin film main member 520 and the second functional layer 540b is the same as in the thin film according to the third embodiment described above.

The first functional layer 530b is made up of a pair of front-side functional layer elements 560a and 560b, that are elliptic sheets laminated at two locations on the front face of the thin film main member 520. The pair of front-side functional layer elements 560a and 560b is configured of a second functional component including an ultraviolet ray protection component (ultraviolet ray absorbing component, ultraviolet ray scattering component). The pair of front-side functional layer elements 560a and 560b is formed on the thin film main member 520 at positions that can be applied to the two discoloration regions 511 formed on the skin in a state of overlaying the discoloration regions 511. In the case of this example, the pair of front-side functional layer elements 560a and 560b overlays a pair of back-side functional layer elements 550c and 550d in entirety, in the front-back direction. Other configurations are the same as in the third embodiment describe above. The forming method of the thin film 50d according to the present embodiment is almost the same as the forming method of the thin film described with reference to Fig. 4.

### [4.1 Effects and Advantages]

According to the thin film 50d of the present embodiment, different effects can be imparted on the discoloration region 511 with a single thin film 50d. That is to say, the thin film 50d can exhibit UV protection effects based on the first functional component and whitening effects based on the second functional component as to the discoloration region 511. The whitening component has a nature of readily deteriorating or denaturing under light. In the case of the present embodiment, the beauty component included in the second functional component deteriorates or denatures less readily due to the UV protection effects based on the first functional component.

### [5. Notes on Embodiments]

Making the shape of the first functional layer and the second functional layer of the thin film in the embodiments to be slightly larger than the shape of the applied face, for example, facilitates dealing with positional deviation when applying the thin film. The first functional component and second functional component are not restricted to one type of functional component, and may be made up of multiple types of functional components.

The first functional layer and the second functional layer do not have to be overlaid in the front-back direction.

The combination of the first and second functional component is not restricted to the above-described embodiments.

In a case where the thin film main member has permeability of functional components, a difference can be created between the time of the functional layer at the skin-side face side of the thin film main member (e.g., second functional layer) permeating the applied face and the time of the functional layer at the outer-side face side (e.g., first functional layer) permeating the applied face. Accordingly, the relation between the time of effects of the functional layer at the skin-side face side being imparted to the applied face and the time of effects of the functional layer at the outer-side face side being imparted to the applied face can be adjusted. For example, in a case where the thin film main member is formed of fibers, the fiber density (in other words, the fineness of the mesh) can be adjusted by forming conditions. Adjusting the fiber density in this way enables the speed of the functional layer at the outer-side face side permeating the thin film main member made of fiber to be adjusted. As a result, the relation between the time of the functional layer at the skin-side face side permeating the skin and the time of the functional layer at the outer-side face side permeating the skin can be optionally adjusted. As a specific example, in a case where determination has been made that the skin is somewhat dry based on measurement results of skin moisture or on user input results, a functional component that softens the skin (e.g., a booster component) is applied to the functional layer at the skin-side face side, and a moisturizing functional component is laminated on the functional layer at the outer-side face side. Accordingly, the functional component of the functional layer at the outer-side face side can be made to permeate the skin, while sufficiently improving the skin by the functional component at the functional layer on the skin-side face side.

Also, in a case where there is a difference in transpiration rate between the functional component included in the first functional layer and the functional component included in the second functional layer, the functional layer containing the functional component that readily transpires is situated at the skin-side face side, and the functional layer containing the functional component that does not readily transpire is situated at the outer-side face side. As a result, transpiration of the functional component that readily transpires can be suppressed, so the effects of the functional components can be exhibited without waste. Examples of functional components that do not readily transpire include so-called makeup components and so forth.

### Industrial Applicability

The thin film applying device according to the present disclosure is particularly useful in usages relating to beauty.

### Reference Signs List

- 100: makeup support system
- 200: image processing device
- 201: illumination unit
- 202: camera
- 203: display unit
- 300: printing device
- 400: user
- 50, 50a, 50c, 50d: thin film
- 511: discoloration region
- 520: thin film main member
- 530, 530a. 530b: first functional layer
- 540, 540a, 540b: second functional layer
- 550a, 550b, 550c, 550d: back-side functional layer elements
- 560a, 560b: front-side functional layer elements

## Claims

1. A thin film that is applied to an applied face of an application object, the thin film comprising:
a thin film main member that has biocompatibility;
a first functional layer including a first functional component laminated on a front face of the thin film main member; and
a second functional layer including a second functional component that differs from the first functional component, layered on a back face of the thin film main member.

2. The thin film according to Claim 1,
wherein the application object is human skin,
and wherein the first functional component and the second functional component differ with regard to cosmetic effects.

3. The thin film according to Claim 1,
wherein, at the time of use, the back face is disposed at the applied face side, and the front face at the opposite side thereof,
wherein the second functional component does not pass through the thin film main member, the first functional component passes through the thin film main member, and both permeate the applied face,
and wherein the thin film main member delays permeation of the first functional component to the applied face more than permeation of the second functional component to the applied face.

4. The thin film according to Claim 1,
wherein, at the time of use, the back face is disposed at the applied face side, and the front face at the opposite side thereof,
wherein the functional component with a lower transpiration rate is laminated on the front face as the first functional component,
and wherein the functional component with a higher transpiration rate is laminated on the back face as the second functional component.

5. The thin film according to Claim 1,
wherein, at the time of use, the back face is disposed at the applied face side, and the front face at the opposite side thereof,
and wherein the first functional component is any one of a color component and an ultraviolet ray protection component.

6. A thin film forming method of forming a thin film to be applied to an applied face of human skin, the method comprising:
a step of obtaining an image including at least the applied face, with the skin as a subject;
a step of extracting shape information relating to the applied face from the image;
a step of printing a first printing material including a first functional component having cosmetic effects on a front face of a thin film main member of the thin film, based on the shape information, thereby forming a first functional layer of the thin film; and
a step of printing a second printing material including a second functional component having cosmetic effects different from those of the first functional component, on a back face of the thin film main member, based on the shape information, thereby forming a second functional layer of the thin film.

7. The thin film forming method according to Claim 6,
wherein, after the step of forming the first functional layer, the first functional layer is subjected to drying processing, and thereafter the step of forming the second functional layer is executed.
